# EUROPEAN PATENT APPLICATION

(11) **EP 4 306 044 A1**
(43) Date of publication of application: **17.01.2024**
(21) Application number: 22185335.1
(22) Date of filing: 15.07.2022
(51) Int. Cl.: A61B 5/024, A61B 5/083, A61B 5/00

(54) **METHOD FOR EVALUATING CARDIAC LOAD AND DEVICE THEREOF**

(71) Applicant: Taoyuan Armed Forces General Hospital, Taoyuan City 32551 (TW)
(72) Inventor: Chan, Jeng-Shyong, 32551 Taoyuan City (TW); Hsiao, Po-Jen, 32551 Taoyuan City (TW); Yang, Chung-Chi, 32551 Taoyuan City (TW); Li, Yuan-Kuei, 32551 Taoyuan City (TW); Chu, Chi-Ming, 114 Taipei City (TW); Chiang, Shang-Lin, 115613 Taipei City (TW); Tsai, Pei-Jan, 114 Taipei City (TW); Wu, Hao-Yi, 114202 Taipei City (TW); Wu, Yi-Syuan, 114 Taipei City (TW)
(74) Representative: Scholz, Volker

(57) **Abstract**

The invention relates to a method for evaluating cardiac load and device thereof; the evaluating device measures the heartbeat number and moving displacement of a test specimen within a fixed time to calculate the displacement per heartbeat, maximum oxygen consumption, anaerobic threshold, and oxygen pulse. This invention uses this immediate, non-invasive evaluating method to make the test specimen evaluate the degree of cardiac load before exercising itself to reduce the occurrence of exercise injury.

## Description

### [FIELD OF THE INVENTION]

This invention relates to a method and device for evaluating cardiac load, particularly an instant, non-invasive evaluating method, and device of cardiac load.

### [BACKGROUND OF THE INVENTION]

A heart is like a pump, responsible for transporting blood to the various organs of the body to provide oxygen and nutrients. The so-called sudden cardiac death refers to the loss of the blood pump function in a heart and the inability to maintain an appropriate cardiac output, resulting in damage to peripheral organs and the sudden death.

The definition of sudden cardiac death is the unanticipated death caused by heart disease, the incidence in men is higher than in women. The average age of occurrence is about 55 to 58 years old, and the incidence increases along with increasing age. For example, the incidence of 50-year-old person is 1/1,000, yet, at the age of 75, the incidence increases to 8/1,000. According to statistics, people who are urgently sent to the hospitals because of myocardial infarction caused by cardiac problems have a survival rate of 10%-30% only.

Moreover, according to statistics, the occurrence of sudden cardiac death is often rapid. Some patients may feel a rapid heartbeat or dizziness when the fatal arrhythmia begins to occur. However, in over half of the cases, there is often no prior warning. Furthermore, when sudden death occurs, if CPR (cardiopulmonary resuscitation) can be performed within 4 minutes or AED shock can be made within 8 minutes, there is a 60%-75% chance to rescue the patient. But, if it takes more than 4 minutes to perform first aid, the survival rate is only 1%.

For this reason, monitoring of cardiac load is necessary in sudden cardiac death prevention. Yet, in addition to that ordinary people need to pay attention to the state of the heart, athletes (or those who need intense physical exercises) need to timely monitor their cardiac load because high-intensity exercise will cause the heart to bear a greater burden due to the increase of cardiac output, and intense exercise also increases blood pressure and puts a heavier load on the heart, which is why athletes are more prone to sudden death.

The current heart monitoring systems can only show the user's heartbeat rater, respiration, blood pressure, moving distance, and location, etc. However, these data cannot provide users with information on the current state of their hearts nor whether the hearts can be loaded for the next exercises or not. Exercising without knowing whether your heart can handle it or not would increases the risk of injury or accidental cardiac death.

Therefore, how to provide a non-invasive device or a method that can immediately know the degree of respective cardiac load is an issue that shall be solved by those skilled in this art.

### [SUMMARY]

The main purpose of this invention is to provide a method for evaluating cardiac load and device thereof, by means of measuring the heartbeat number and moving displacement of the test specimen within a fixed time by the evaluating device, it can calculate the displacement per heartbeat, maximum oxygen consumption, anaerobic threshold, and oxygen pulse. This immediate, non-invasive evaluating method enables the test specimen to evaluate the degree of his/her cardiac load before exercising, which can reduce the occurrence of exercise injury.

To achieve the above purpose, this invention reveals a method for evaluating cardiac load. The steps comprise: measuring a heartbeat number of a test specimen within a period with a heartbeat rate detection unit, and measuring a displacement of the test specimen within the period with a distance measuring unit; the heartbeat rate detection unit and the distance measuring unit transmit the heartbeat number and the displacement to an operation unit respectively; the operation unit obtains a displacement per heartbeat according to the heartbeat number and displacement, the calculation is: the displacement per heartbeat equal to the displacement divided by the heartbeat number; when the displacement per heartbeat is greater than a first preset value or less than a second preset value, the operation unit transmits a warning message and the displacement per heartbeat to a display unit; and the display unit displays the warning message and the displacement per heartbeat, and warns the test specimen; wherein the displacement unit is centimeter (cm), the heartbeat number is the heartbeats per second, the heartbeat rate detection unit measures the heartbeat number of the test specimen by measuring the heartbeats or arterial beats of the test specimen .

This invention provides an embodiment, the content of which is a method for evaluating cardiac load, in which in the step that the operation unit obtains a displacement per heartbeat according to the heartbeat number and the displacement t, the calculation is: the displacement per heartbeat equal to the displacement divided by the heartbeat number, it also comprise the following steps: the operation unit obtains an hourly speed according to the displacement and the period, and obtains a normalized displacement per heartbeat within 3-km distance according to the displacement per heartbeat and the hourly speed, the calculation is: the normalized displacement per heartbeat within 3-km distance equal to the displacement per heartbeat multiplying (3 divided by the hourly speed); the operation unit transmits the normalized displacement per heartbeat within 3-km distance to the display unit; and the display unit displays the normalized displacement per heartbeat within 3-km distance.

Also, this invention reveals a device for evaluating cardiac load, which comprises: a heartbeat rate detection unit used to measure a heartbeat number of a test specimen; a distance detection unit used to measure a displacement of the test specimen; an operation unit used to receive the heartbeat number and the displacement, and calculate a displacement per heartbeat according to the heartbeat number and the displacement, the calculation is: the displacement per heartbeat equal to the displacement divided by the heartbeat number; and a display unit used receive the displacement per heartbeat sent by the operation unit and to display the displacement per heartbeat; wherein when the displacement per heartbeat is greater than a first preset value or less than a second preset value, the operation unit further transmits a warning message to the display unit.

This invention provides an embodiment, the content of which is a device for evaluating cardiac load, in which the operation unit further obtains an hourly speed according to the displacement and the period, and a normalized displacement per heartbeat within 3-km distance according to the displacement per heartbeat and the hourly speed, the calculation is: the normalized displacement per heartbeat within 3-km distance equal to the displacement per heartbeat multiplying (3 divided by hourly speed),and the operation unit further transmits the normalized displacement per heartbeat within 3-km distance to the display unit displaying the normalized displacement per heartbeat within 3-km distance.

This invention provides an embodiment, which is a device for evaluating cardiac load, in which the evaluating device further comprises a positioning unit used to locate and obtain the position of the test specimen.

Furthermore, this invention reveals a method for evaluating cardiac load, the steps comprise: measuring a heartbeat number of a test specimen within a period with a heartbeat rate detection unit, and measuring a displacement of the test specimen within the period with a distance measuring unit; the heartbeat rate detection unit and the distance measuring unit transmit the heartbeat number and the displacement to an operation unit respectively; the operation unit obtains a maximum oxygen consumption according to the heartbeat number and the displacement, the calculation is: the maximum oxygen consumption equal to the displacement divided by (the heartbeat number multiplying K), wherein 0.71 ≦K≦3.63; when the maximum oxygen consumption is greater than a third preset value or less than a fourth preset value, the operation unit transmits a warning message and the maximum oxygen consumption to a display unit; and the display unit displays the warning message and the maximum oxygen consumption and warns the test specimen; wherein the heartbeat number is the heartbeats per second, the heartbeat rate detection unit measures the heartbeat number of the test specimen by measuring the heartbeats or arterial beats of the test specimen.

Furthermore, this invention reveals a device for evaluating cardiac load, which comprises: a heartbeat rate detection unit used to measure a heartbeat number of a test specimen; a distance measuring unit used to measure a displacement of the test specimen; an operation unit used to receive the heartbeat number and the displacement, and calculate a maximum oxygen consumption according to the heartbeat number and the displacement, the calculation is: the maximum oxygen consumption equal to the displacement divided by (the heartbeat number multiplying K), where 0.71 ≦ K ≦ 3.63; and a display unit used to receive the maximum oxygen consumption sent by the operation unit and to display the maximum oxygen consumption; wherein when the maximum oxygen consumption is greater than a third preset value or less than a fourth preset value, the operation unit further transmits a warning message to the display unit.

This invention provides an embodiment, which is a device for evaluating cardiac load, in which the evaluating device further comprises a positioning unit used to locate and obtain the position of the test specimen.

Furthermore, this invention reveals a method for evaluating cardiac load, the steps comprises: measuring a heartbeat number of a test specimen within a period with a heartbeat rate detection unit, and measuring a displacement of the test specimen within the period with a distance measuring unit; the heartbeat rate detection unit and the distance measuring unit transmit the heartbeat number and the displacement to an operation unit respectively; the operation unit obtains an anaerobic threshold according to the heartbeat number and the displacement, the calculation is: the anaerobic threshold equal to the displacement divided by (the heartbeat number multiplying M), wherein 20.5 ≦M≦ 112.07; when the anaerobic threshold is greater than a fifth preset value or less than a sixth preset value, the operation unit transmits a warning message and the anaerobic threshold to a display unit; and the display unit displays the warning message and the anaerobic threshold and warns the test specimen; wherein the heartbeat rate is the heartbeats per second; the heartbeat rate detection unit measures the heartbeat number of the test specimen by measuring his/her heartbeats or arterial beats.

Furthermore, this invention reveals a device for evaluating cardiac load, which comprises: a heartbeat rate detection unit used to measure a heartbeat number of a test specimen; a distance measuring unit used to measure a displacement of the test specimen; an operation unit used to receive the heartbeat number and the displacement and calculate an anaerobic threshold according to the heartbeat number and the displacement, the calculation is: the anaerobic threshold equal to the displacement divided by (the heartbeat number multiplying M), wherein 20.5 ≦M≦ 112.07; and a display unit used to receive the anaerobic threshold sent by the operation unit, and to display the anaerobic threshold; wherein when the anaerobic threshold is greater than a fifth preset value or less than a sixth preset value, the operation unit further transmits a warning message to the display unit.

Furthermore, this invention reveals a method for evaluating cardiac load, the steps comprise: measuring a heartbeat number of a test specimen within a period with a heartbeat rate detection unit, and measuring a displacement of the test specimen within the period with a distance measuring unit; the heartbeat rate detection unit and the distance measuring unit transmit the heartbeat number and the displacement to an operation unit respectively; the operation unit obtains an oxygen pulse according to the heartbeat number and the displacement, the calculation is: the oxygen pulse equal to the displacement divided by (the heartbeat number multiplying N), wherein 1.36≦N≦ 5.58; when the oxygen pulse is greater than a seventh default value or less than an eighth default value, the operation unit transmits a warning message and the oxygen pulse to a display unit; and the display unit displays the warning message and the oxygen pulse and warns the test specimen; wherein the heartbeat number is the heartbeats per second, and the heartbeat rate detection unit measures the heartbeat number of the test specimen by measuring the heartbeats or arterial beats of the test specimen.

Furthermore, this invention reveals a device for evaluating cardiac load, which comprises: a heartbeat rate detection unit used to measure a heartbeat number of a test specimen; a distance measuring unit used to measure a displacement of the test specimen; an operation unit used to receive the heartbeat number and the displacement, and calculate the oxygen pulse according to the heartbeat number and the displacement, the calculation is: the oxygen pulse equal to the displacement divided by (the heartbeat number multiplying N), where 1.36≦N≦5.58; and a display unit used to receive the oxygen pulse sent by the operation unit and to display the oxygen pulse; wherein when the oxygen pulse is greater than a seventh preset value or less than an eighth preset value, the operation unit further transmits a warning message to the display unit.

### [BRIEF DESCRIPTION OF THE DRAWINGS]

Fig. 1A: The flowchart in the first embodiment of this invention;
Fig. 1B: The flowchart in the first embodiment of this invention;
Fig. 2: The flowchart in the second embodiment of this invention;
Fig. 3: The flowchart in the third embodiment of this invention; and
Fig. 4: The flowchart in the fourth embodiment of this invention.

### [DETAILED DESCRIPTION]

To enable the Review Committee members to have a deeper realization and understanding of the features and functions of this invention, we hereby put the embodiment and detailed explanation in below:

Since not knowing about whether one's own heart can withstand the load under an exercising status would increase the risk of injury or accidental cardiac death. Accordingly, this invention proposes an evaluating method and an evaluating device of cardiac load to solve the problems caused by the prior art.

The characteristics, structure and methods included in the evaluating method and device of cardiac load proposed in this invention is further described in below:

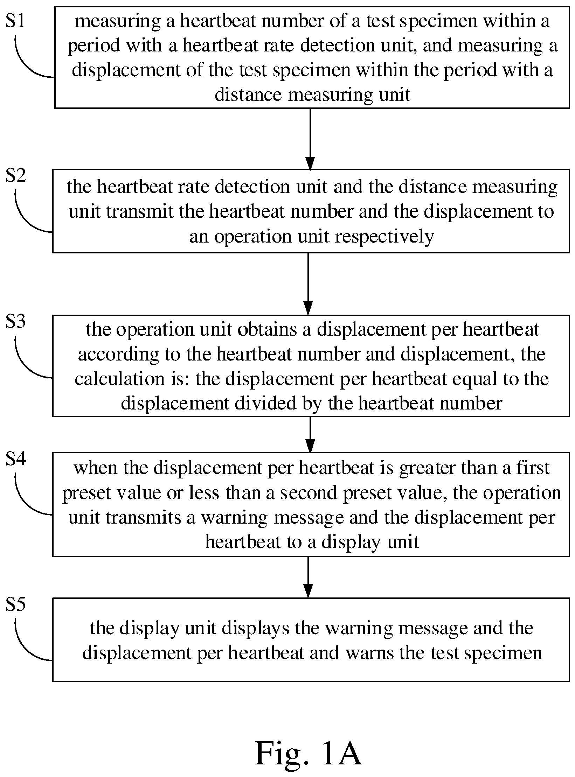

Referring to Fig. 1A, which is the flowchart in the first embodiment of this invention. As shown in Fig. 1A, a method for evaluating cardiac load, the steps comprise:
S1: measuring a heartbeat number of a test specimen within a period with a heartbeat rate detection unit, and measuring a displacement of the test specimen within the period with a distance measuring unit;
S2: the heartbeat rate detection unit and the distance measuring unit transmit the heartbeat number and the displacement to an operation unit respectively;
S3: the operation unit obtains a displacement per heartbeat according to the heartbeat number and displacement, the calculation is: the displacement per heartbeat equal to the displacement divided by the heartbeat number;
S4: when the displacement per heartbeat is greater than a first preset value or less than a second preset value, the operation unit transmits a warning message and the displacement per heartbeat to a display unit; and
S5: the display unit displays the warning message and the displacement per heartbeat and warns the test specimen.

As shown in step S1, measuring a heartbeat number (heartbeats per sec) of a test specimen within a period by a heartbeat rate detection unit is the measurement of heartbeats or arterial beats of the test specimen, the measuring method is unlimited; for example, measuring the electrical impulses generated by each heartbeat cycle, the blood vessel pressure that changes with the heartbeat, or the blood flow in the micro-vessels of the finger or earlobe, etc.; and measuring a distance (unit: cm) of the test specimen by a distance measuring unit within the same period.

In which the heartbeat rate detection unit and the distance measuring unit are worn on the test specimen, and the wearing method is not limited to any method, such as by the watch type, attached type or wearable type, etc. Moreover, the heartbeat rate detection unit and the distance measuring unit are connected in a wireless or wired manner, such as: Bluetooth, ZigBee, wireless network (Wi-Fi), or very high frequency (VHF).

The distance measuring unit is made by an accelerometer of any form, such as: the three-axis accelerometer, nine-axis accelerometer, sixteen-axis accelerometer, and gravity sensor (G-sensor).

Continuing to step S2, the heartbeat rate detection unit and the distance measuring unit respectively transmit the heartbeat number and the displacement to an operation unit. And as shown in step S3, the operation unit makes calculation according to the heartbeat number and the displacement to obtain the displacement per heartbeat (CMPB); the calculation is the displacement per heartbeat = the displacement / the heartbeat number.

Also, as shown in step S4, when the displacement per heartbeat is greater than a first preset value or less than a second preset value, the operation unit transmits a warning message and the displacement per heartbeat to a display unit. The first preset value and the second preset value are obtained by the standard specification and statistical experience of conventional maximum oxygen consumption. The first preset value is 65 and the second preset value is 40.

The stages of the displacement per heartbeat are: When CMPB<35, it is grade 3 or 4 of heart failure; when CMPB<40, it is grade 1 or 2 of heart failure; when CMPB=45, it is at healthy condition; when CMPB=50, it is at the condition of a young person; when CMPB = 55, it is under the condition of liver-healthy-person; when CMPB = 60, it is under the athlete condition; when CMPB >65, it is at grade 1 or grade 2 of heart failure; and when CMPB>70, it is at grade 3 or grade 4 of heart failure.

Further, the warning message of this invention is that after the displacement per heartbeat is greater than the first preset value or less than the second preset value, the warning message will be issued in different degrees according to the values of the displacement per heartbeat.

Finally, as shown in step S5, the display unit displays the warning message and the displacement per heartbeat and warns the test specimen; it will issue different degrees of warning message according to the value of the displacement per heartbeat to warn the test specimen, letting the test specimen know his/her own cardiac load status.

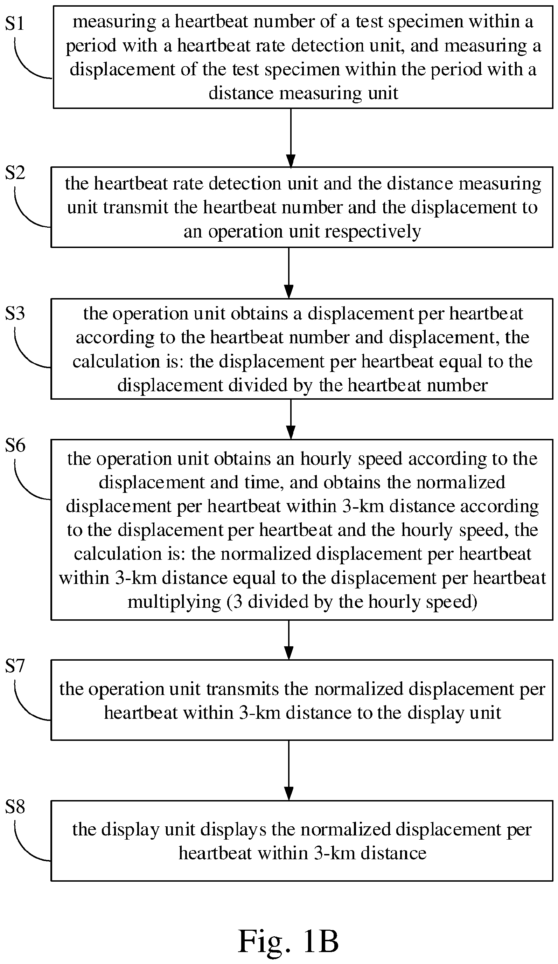

This invention further comprises steps S6~S8 after step S3 (as shown in Fig. 1B, which is the flowchart in the first embodiment of this invention):
S6: the operation unit obtains an hourly speed according to the displacement and time, and obtains the normalized displacement per heartbeat within 3-km distance according to the displacement per heartbeat and the hourly speed, the calculation is: the normalized displacement per heartbeat within 3-km distance equal to the displacement per heartbeat multiplying (3 divided by the hourly speed);
S7: the operation unit transmits the normalized displacement per heartbeat within 3-km distance to the display unit; and
S8: the display unit displays the normalized displacement per heartbeat within 3-km distance.

According to the literature in 2011 (Studenski et al., 2011), if the age- and gender-adjusted walking speed is greater than about 0.8 m/s (about 2.88 km/h), the life expectancy is greater than the median (Studenski et al., 2011). Therefore, the normalized speed is set at about 3 kilometers per hour; through normalization process, the speed is uniformly converted to 3 kilometers per hour.

And as shown in step S6, the operation unit obtains an hourly speed according to the displacement and the period, and obtains a normalized displacement per heartbeat within 3-km distance according to the displacement per heartbeat and the hourly speed; the calculation is the normalized displacement per heartbeat within 3-km distance = the displacement per heartbeat x (3/ the hourly speed).

Continuing to step S7, the operation unit transmits the normalized displacement per heartbeat within 3-km distance to the display unit. The closer the normalized displacement per heartbeat within 3-km distance is to 50, the more normal it is. On the contrary, the more it deviates from 50, the more it needs to pay attention to the cardiac load condition.

Finally, as shown in step S8, the display unit displays the normalized displacement per heartbeat within 3-km distance, in which the closer the normalized displacement per heartbeat within 3-km distance is to 50, the more normal it is. On the contrary, the more it deviates from 50, the more attention should be paid own the self-cardiac-load state of test specimen. Through the value of the normalized displacement per heartbeat within 3-km distance, the test specimen can know his/her own cardiac load state.

The evaluating method (two types) of the aforesaid first embodiment can be used not only for cardiac monitoring in daily life; it also can be used in monitoring the cardiac load state during any exercises, such as: jogging, fast running, marathon, biking, and swimming. It also can be used for a pre-exercise detection.

Also, the evaluating device for the evaluating method of the first embodiment is as follows:

The device for evaluating cardiac load of this invention comprise: The heartbeat rate detection unit, the distance measuring unit, the operation unit, and the display unit.

The heartbeat rate detection unit is used to measure the heartbeat number of the test specimen within the period, and the distance measuring unit is used to measure the displacement of the test specimen within the same period. After the operation unit receives the heartbeat number and the displacement, the displacement per heartbeat is calculated according to the heartbeat number and the displacement. The calculation is the displacement per heartbeat = the displacement / the heartbeat number (or further according to the displacement and the period obtained the hourly speed; following the displacement per heartbeat and the hourly speed, it can obtain the normalized displacement per heartbeat within 3-km distance; the calculation is the normalized displacement per heartbeat within 3-km distance = the displacement per heartbeat x (3 /the hourly speed)).

When obtaining the displacement per heartbeat (or the normalized displacement per heartbeat within 3-km distance), the operation unit sends the displacement per heartbeat (or the normalized displacement per heartbeat within 3-km distance) to the display unit to display the displacement per heartbeat (or the normalized displacement per heartbeat within 3-km distance) and inform it to the test specimen. When the displacement per heartbeat is greater than the first preset value or less than the second preset value, the operation unit further transmits the warning message to the display unit.

The evaluating device of the cardiac load in this invention further includes a positioning unit to locate and obtain the position of the test specimen. Furthermore, when the test specimen is exercising or under physical training, if the displacement per heartbeat (or the normalized displacement per heartbeat within 3-km distance) does not meet the standard (the displacement per heartbeat is greater than the first preset value or less than the second preset value) and the injured test specimen is unable to contact with outside, the positioning unit can be used to search for the location of the test specimen. The positioning unit can be in any form, such as the Global Positioning System (GPS).

Furthermore, the evaluating device of this invention further includes a memory unit, which can store the historic displacement per heartbeat (or the normalized displacement per heartbeat within 3-km distance) of the test specimen. The memory unit can be in any form, such as the flash memory. The test specimen can understand and observe the state of his/her cardiac load in different time periods through the memory unit.

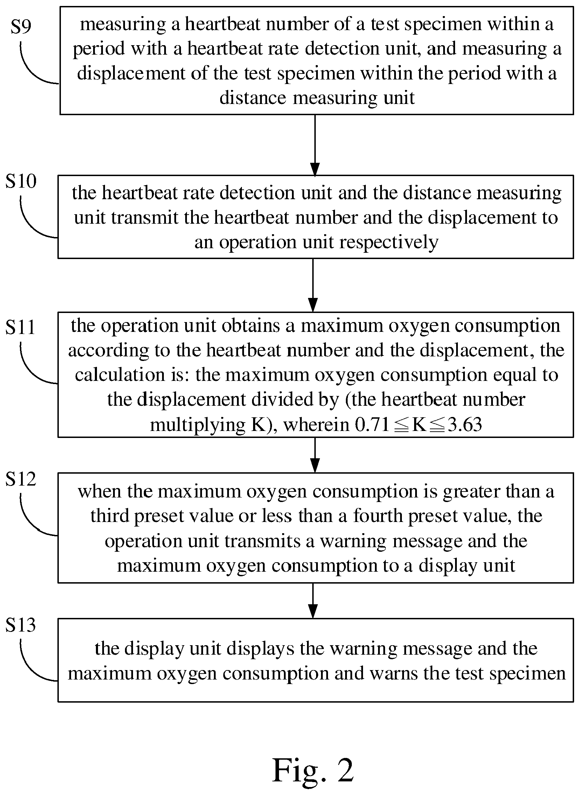

Continuing to above, referring to Fig. 2, which is a flow chart of the flowchart in the second embodiment of this invention. As shown in Fig. 2, a method for evaluating cardiac load, the steps comprise:
S9: measuring a heartbeat number of a test specimen within a period with a heartbeat rate detection unit, and measuring a displacement of the test specimen within the period with a distance measuring unit;
S10: the heartbeat rate detection unit and the distance measuring unit transmit the heartbeat number and the displacement to an operation unit respectively;
S11: the operation unit obtains a maximum oxygen consumption according to the heartbeat number and the displacement, the calculation is: the maximum oxygen consumption equal to the displacement divided by (the heartbeat number multiplying K), wherein 0.71≦K≦3.63;
S12: when the maximum oxygen consumption is greater than a third preset value or less than a fourth preset value, the operation unit transmits a warning message and the maximum oxygen consumption to a display unit; and
S13: the display unit displays the warning message and the maximum oxygen consumption and warns the test specimen.

As shown in step S9, the heartbeat number (heartbeats per second) of the test specimen is measured by the heartbeat rate detection unit within the period, which is to measure the heartbeats or arterial beats of the test specimen; the method is not limited, such as measuring the electrical pulse generated by each heartbeat cycle, the blood vessel pressure that changes with the heartbeat, or the blood flow of the micro-vessels of the finger or earlobe, etc., and measuring the displacement (unit: cm) of the test specimen by using the distance measuring unit within the same period.

The heartbeat rate detection unit and the distance measuring unit are worn on the test specimen, and the wearing method is unlimited, such as by the watch type, attached type, or wearable type, etc. Moreover, the heartbeat rate detection unit and the distance measuring unit are connected in a wireless or wired manner, such as: Bluetooth, ZigBee, wireless network (Wi-Fi), or very high frequency (VHF).

The distance measuring unit is made by accelerometers in any forms, such as: the three-axis accelerometer, nine-axis accelerometer, sixteen-axis accelerometer, and gravity sensor (G-sensor).

Then, as shown in step S10, the heartbeat rate detection unit and the distance measuring unit transmit the heartbeat number and the displacement to the operation unit respectively. As shown in step S11, this operation unit makes calculation according to numerical values of heartbeat number and displacement, and obtains a maximum oxygen consumption (VO2 max); the calculation is: The maximum oxygen consumption = the displacement / (the heartbeat number x K), where 0.71≦K≦3.63, where K is a constant obtained from statistical data, the satisfactory interval (between 0.71 and 3.63) varies from individual differences among populations.

Also, as shown in step S12, when the maximum oxygen consumption is greater than a third preset value or less than a fourth preset value, the operation unit transmits the warning message and the maximum oxygen consumption to the display unit. The third preset value and the fourth preset value are obtained by the standard specification and statistical experience of conventional maximum oxygen consumption. The third preset value is 91.55, and the fourth preset value is 11.02.

When the maximum oxygen consumption is between 11.02 and 17.91 and between 56.34 and 91.55, the display unit will show the value to notify the test specimen to pay attention to. If it is greater than 91.55 or less than 11.02, the operation unit sends the warning message and the maximum oxygen consumption to the display unit.

Furthermore, after the maximum oxygen consumption is greater than the third preset value or less than the fourth preset value, the warning messages of this invention are sent out with different degrees according to the value of the maximum oxygen consumption.

Finally, as shown in step S13, the display unit displays the warning message and the maximum oxygen consumption and warns the test specimen; it will issue different degrees of warning messages to warn the test specimen according to the value of the maximum oxygen consumption, letting the test specimen know his/her state of cardiac load.

In addition to monitoring the cardiac state in daily life, this evaluating method also can be used to monitor the cardiac load state during any exercise, such as: jogging, fast running, marathon, biking, and swimming. It also can be used as the pre-detection before exercise.

Also, the evaluating device of the evaluating method in the second embodiment is as follows:
The device for evaluating cardiac load, which comprises: the heartbeat rate detection unit, the distance measuring unit, the operation unit, and the display unit.

The heartbeat rate detection unit is used to measure the heartbeat number of the test specimen within the period, and the distance measuring unit is used to measure the displacement of the test specimen within the same period. After receiving the heartbeat number and the displacement, the operation unit calculates the maximum oxygen consumption according to the heartbeat number and the displacement; the calculation is: the maximum oxygen consumption = the displacement / (the heartbeat number x K), where 0.71≦K≦3.63, K is a constant obtained from statistical values, the satisfactory interval (between 0.71 and 3.63) varies from individual differences among populations.

When the maximum oxygen consumption is obtained, it is sent to the display unit from the operation unit and is displayed to inform the test specimen. Yet, when the maximum oxygen consumption is greater than the third preset value or less than the fourth preset value, the operation unit further transmits the warning message to the display unit.

The device for evaluating cardiac load in this invention further comprises the positioning unit to locate and obtain the position of the test specimen. Furthermore, when the test specimen is exercising or under physical training, if the maximum oxygen consumption does not meet the standard (the maximum oxygen consumption is greater than the third preset value or less than the fourth preset value), and the injured test specimen is unable to contact with outside, the positioning unit can be used to search for the location of the test specimen. The positioning unit can be in any form, such as the Global Positioning System (GPS).

Meanwhile, the device for evaluating cardiac load in this invention further comprises the memory unit, which can store the historic maximum oxygen consumption data of the test specimen. The memory unit can be in any form, such as the flash memory. The test specimen can understand and observe the state of his/her cardiac load in different time periods through the memory unit.

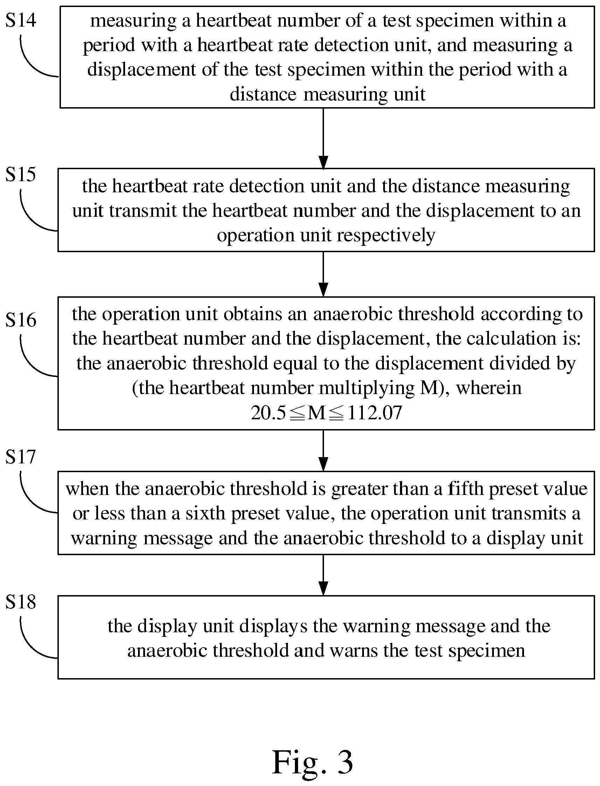

Continuing to above, referring to Fig. 3, which is a flowchart of the third embodiment in this invention. As shown in Fig. 3, a method for evaluating cardiac load, the steps comprise :
S14: measuring a heartbeat number of a test specimen within a period with a heartbeat rate detection unit, and measuring a displacement of the test specimen within the period with a distance measuring unit;
S15: the heartbeat rate detection unit and the distance measuring unit transmit the heartbeat number and the displacement to an operation unit respectively;
S16: the operation unit obtains an anaerobic threshold according to the heartbeat number and the displacement, the calculation is: the anaerobic threshold equal to the displacement divided by (the heartbeat number multiplying M), wherein 20.5≦M≦112.07;
S17: when the anaerobic threshold is greater than a fifth preset value or less than a sixth preset value, the operation unit transmits a warning message and the anaerobic threshold to a display unit; and
S18: the display unit displays the warning message and the anaerobic threshold and warns the test specimen.

As shown in step S14, it uses the heartbeat rate detection unit to measure the heartbeat number (heartbeats per second) of the test specimen within the period, which is to measure the heartbeats or arterial beats of the test specimen, the method is not limited, such as measuring the electrical pulse generated by each heartbeat cycle, the blood vessel pressure that changes with the heartbeat, or the blood flow of the micro-vessels of the finger or earlobe, etc., and measuring the displacement (unit: cm) of the test specimen by using the distance measuring unit.

The heartbeat rate detection unit and the distance measuring unit are worn on the test specimen, and the wearing method is unlimited, such as by the watch type, attached type, or wearable type, etc. Moreover, the heartbeat rate detection unit and the distance measuring unit are connected in a wireless or wired manner, such as: Bluetooth, ZigBee, wireless network (Wi-Fi), or very high frequency (VHF).

The distance measuring unit is made by accelerometers in any forms, such as: the three-axis accelerometer, nine-axis accelerometer, sixteen-axis accelerometer, and gravity sensor (G-sensor). Then, as shown in step S16, the operation unit makes calculation according to the heartbeat number and displacement, and obtains an anaerobic threshold; the calculation is: the anaerobic threshold = the displacement / (the heartbeat number x M), where 20.5≦M≦112.07, M is a constant obtained from statistical values, the satisfactory interval (between 20.5 and 112.07) varies from individual differences among populations.

Furthermore, as shown in step S17, when the anaerobic threshold is greater than a fifth preset value or less than a sixth preset value, the operation unit transmits the warning message and the anaerobic threshold to the display unit. The fifth preset value and the sixth preset value are obtained through the standard specification and statistical experience of conventional maximum oxygen consumption. The fifth preset value is 3.17, and the sixth preset value is 0.35.

When the anaerobic threshold is between 0.35~0.57 and 1.95~3.17, the display unit will display its value letting the test specimen pay attention about. If it is greater than 3.17 or less than 0.35, the operation unit transmits the warning message and the anaerobic threshold to the display unit.

Finally, as shown in step S18, the display unit displays the warning message and the anaerobic threshold and warns the test specimen, which will issue different degrees of warning messages to warn the test specimen according to the value of the anaerobic threshold, letting the test specimen know his/her state of cardiac load.

In addition to cardiac monitoring in daily life, this evaluating method can also be used to monitor the state of cardiac load during various physical exercises, such as jogging, fast running, marathon, biking, and swimming. It also can be used as the pre-detection before any exercises.

Further, the evaluating device of the evaluating method in the third embodiment is as follows:
The device for evaluating cardiac load in this invention comprises: The heartbeat rate detection unit, the distance measuring unit, the operation unit, and the display unit.

The heartbeat rate detection unit is used to measure the heartbeat number of the test specimen within the period; and the distance measuring unit is used to measure the displacement of the test specimen within the same period. After the operation unit has received the heartbeat number and the displacement, it calculates the anaerobic threshold according to the heartbeat number and the displacement. The calculation is: the anaerobic threshold = the displacement / (the heartbeat number x M), where 20.5≦M ≦112.07, M is a constant derived from statistical values, the satisfactory interval (between 20.5 and 112.07) varies from individual differences among populations.

After obtaining the anaerobic threshold, the operation unit transmits it to the display unit, where the anaerobic threshold is displayed to inform the test specimen. If the anaerobic threshold is greater than the fifth preset value or less than the sixth preset value, the operation unit further transmits the warning message to the display unit.

The device for evaluating cardiac load in this invention further comprises the positioning unit to locate and obtain the position of the test specimen. Furthermore, when the test specimen is exercising or under physical training, if the anaerobic threshold does not meet the standard (the anaerobic threshold is greater than the fifth preset value or less than the sixth preset value), and the injured test specimen is unable to contact with outside, the positioning unit can be used to search for the location of the test specimen. The positioning unit can be in any form, such as the Global Positioning System (GPS).

Also, the device for evaluating cardiac load in this invention further comprises the memory unit that can store the historic anaerobic threshold data of the test specimen. The memory unit can be in any form, such as the flash memory. The test specimen can understand and observe the state of his/her cardiac load in different time periods through the memory unit.

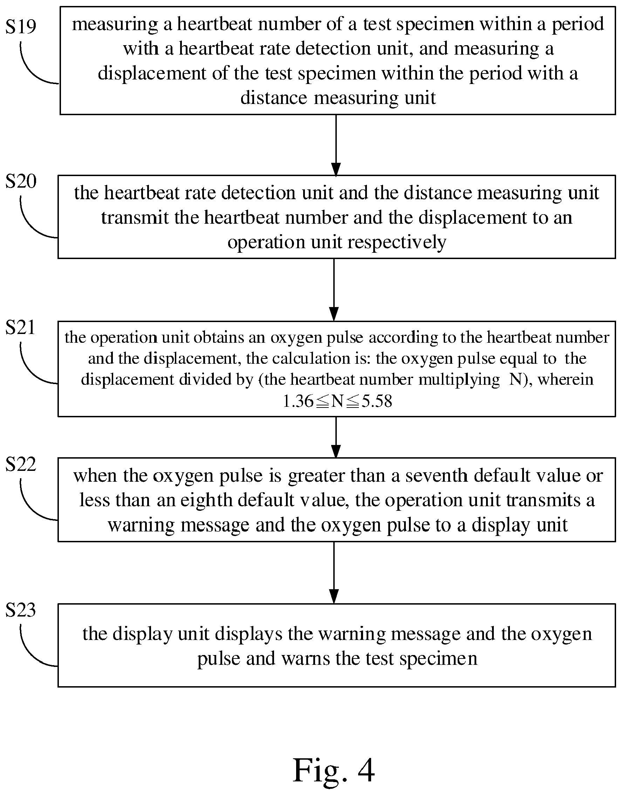

Furthermore, referring to Fig. 4, which is a flowchart of the fourth embodiment in this invention. As shown in Fig. 4, it is a method for evaluating cardiac load, the steps comprise :
S19: measuring a heartbeat number of a test specimen within a period with a heartbeat rate detection unit, and measuring a displacement of the test specimen within the period with a distance measuring unit;
S20: the heartbeat rate detection unit and the distance measuring unit transmit the heartbeat number and the displacement to an operation unit respectively;
S21:the operation unit obtains an oxygen pulse according to the heartbeat number and the displacement, the calculation is: the oxygen pulse equal to the displacement divided by (the heartbeat number multiplying N), wherein 1.36≦N≦5.58;
S22: when the oxygen pulse is greater than a seventh default value or less than an eighth default value, the operation unit transmits a warning message and the oxygen pulse to a display unit; and
S23: the display unit displays the warning message and the oxygen pulse and warns the test specimen.

As shown in step S19, using the heartbeat rate detection unit to measure the heartbeat rate (heartbeats per second) of the test specimen within the period, which is to measure the heartbeats or arterial beats of the test specimen; the measuring method is unlimited; for example, measuring the electrical impulses generated by each heartbeat cycle, the blood vessel pressure that changes with the heartbeat, or the blood flow in the micro-vessels of the finger or earlobe, etc.; and simultaneously measuring a distance (unit: cm) of the test specimen by a distance measuring unit within the period.

The heartbeat rate detection unit and the distance measuring unit are worn on the test specimen, and the wearing method is unlimited, such as by the watch type, attached type, or wearable type, etc. Moreover, the heartbeat rate detection unit and the distance measuring unit are connected in a wireless or wired manner, such as: Bluetooth, ZigBee, wireless network (Wi-Fi), or very high frequency (VHF).

The distance measuring unit is made by accelerometers in any forms, such as: the three-axis accelerometer, nine-axis accelerometer, sixteen-axis accelerometer, and gravity sensor (G-sensor). Then, as shown in step S20, the heartbeat rate detection unit and the distance measuring unit transmit the heartbeat number and the displacement to the operation unit respectively. And as shown in step S21, the operation unit makes calculation according to the heartbeat number and the displacement and obtains an oxygen pulse; the calculation is: the oxygen pulse = the displacement / (the heartbeat number x N), where 1.36≦N≦5.58, where N is a constant obtained from statistical values, the satisfactory interval (between 1.36 and 5.58) varies from individual differences among populations.

Furthermore, as shown in step S22, if the oxygen pulse is greater than a seventh preset value or less than an eighth preset value, the operation unit transmits the warning message and the oxygen pulse to the display unit. The seventh preset value and the eighth preset value are obtained through the standard specification and statistical experience of conventional maximum oxygen consumption. The seventh preset value is 47.79, and the eighth preset value is 7.168.

When the oxygen pulse is between 7.168~11.65 and 29.41~47.7, the display unit will display the value to make the test specimen pay attention. If it is greater than 3.17 or less than 0.35, the operation unit transmits the warning message and the oxygen pulse to the display unit.

Finally, as shown in step S23, the display unit displays the warning message and the oxygen pulse and warns the test specimen, which will issue different degrees of the warning messages to warn the test specimen according to the value of oxygen pulse, letting the test specimen know his/her state of cardiac load.

In addition to cardiac monitoring in daily life, this evaluating method can also be used to monitor the cardiac load state during any exercises, such as: jogging, fast running, marathon, biking, and swimming. It also can be used as the detection before making any exercise.

Also, the evaluating device of the evaluating method in the fourth embodiment is as follows:
The device for evaluating cardiac load in this invention includes: The heartbeat rate detection unit, the distance measuring unit, the operation unit, and the display unit.

The heartbeat rate detection unit is used to measure the heartbeat number of the test specimen within the period; the distance measuring unit is used to measure the displacement of the test specimen within the same period. After the operation unit has received the heartbeat number and the displacement, it calculates the oxygen pulse according to the heartbeat number and the displacement. The calculation is: the oxygen pulse = the displacement / (the heartbeat number x N), where 1.36≦N≦5.58, where N is a constant derived from statistical values, the satisfactory interval (between 1.36 and 5.58) varies from individual differences among populations.

After the oxygen pulse is obtained, the operation unit transmits it to the display unit to display the oxygen pulse and inform the test specimen. If the oxygen pulse is larger than the seventh preset value or smaller than the eighth preset value, the operation unit further transmits the warning message to the display unit.

The device for evaluating cardiac load in this invention further includes the positioning unit to locate and obtain the position of the test specimen. Furthermore, when the test specimen is exercising or under physical training, if the oxygen pulse does not meet the standard (the oxygen pulse is greater than the seventh preset value or less than the eighth preset value), and the injured test specimen is unable to contact with outside, the positioning unit can be used to search for the location of the test specimen. The positioning unit can be in any form, such as the Global Positioning System (GPS).

Also, the device for evaluating cardiac load in this invention further comprises the memory unit, which can store the historic oxygen pulse data of the test specimen. The memory unit can be in any form, such as the flash memory. The test specimen can understand and observe the state of his/her cardiac load in different time periods through the memory unit.

Based on the same theory, this invention is also applicable in monitoring the state of primer (e.g., engine) of a moving object (e.g., a car). In another embodiment, a method for monitoring the state of primer of a moving object includes: The operation unit obtains a load index according to the moving distance of the moving object and the rotational speed of the primer; the calculation equation of the load index is: The loading index = the moving distance of moving object / the speed of primer.

Cardiac Rehabilitation (CR) has been an important non-invasive treatment after the treatment of cardiovascular disease or cardiac surgery, which can improve the cardiopulmonary function of patient.

The method of subsequent evaluation of rehabilitation effect is the cardiopulmonary function test. Through the physical exercise stress test, we can understand the patient's maximum cardiorespiratory endurance and exercise intensity. However, the current cardiopulmonary function tests all must be measured by professional instruments in hospitals, which consume lots of time and money.

However, the measurement of displacement per heartbeat (Centimeters Per Beat, CMPB), normalized displacement per heartbeat within 3-km distance (CMPB3), anaerobic threshold, oxygen pulse and maximum oxygen consumption in this invention can be used easily to know the effect of cardiac rehabilitation.

After 12 weeks of cardiac rehabilitation (twice a week, 20 to 40 minutes of heavy aerobic exercise and a 70% heart rate reserve), the physiological parameters are shown in Table 1 below:

**Table 1. Data table of displacement per heartbeat taken before and after the cardiac rehabilitation**

| | Before rehab. | After rehab. | Difference | p-value^{a} | p-value^{b} |
|---|---|---|---|---|---|
| Heartbeats_ 1 min after starting | 88.449±9.113 | 94.927±11.235 | 6.478±8.798 | 0.099 | 0.091 |
| Heartbeats_ 2 min after starting | 88.850±8.353 | 95.442±10.852 | 6.591±8.194 | 0.077 | 0.028 |
| Heartbeats_ 3 min after starting | 89.121±8.132 | 95.934±11.214 | 6.813±8.789 | 0.086 | 0.043 |
| Heartbeats_ 4 min after starting | 89.328±8.283 | 96.318±11.232 | 6.990±9.020 | 0.086 | 0.043 |
| Heartbeats_ 5 min after starting | 89.367±8.157 | 96.421±11.197 | 7.055±9.023 | 0.084 | 0.043 |
| Heartbeats_ 6 min after starting | 89.286±8.104 | 96.592±11.142 | 7.306±9.073 | 0.077 | 0.028 |
| Hourly speed_1 min after starting | 1.663±0.342 | 2.066±0.405 | 0.403±0.471 | 0.064 | 0.063 |
| Hourly speed_2 min after starting | 1.709±0.232 | 2.066±0.420 | 0.357±0.372 | 0.044 | 0.063 |
| Hourly speed_3 min after starting | 1.728±0.208 | 2.069±0.409 | 0.341±0.370 | 0.051 | 0.063 |
| Hourly speed_4 min after starting | 1.730±0.203 | 2.081±0.414 | 0.351±0.357 | 0.041 | 0.063 |
| Hourly speed_5 min after starting | 1.739±0.215 | 2.076±0.410 | 0.338±0.353 | 0.045 | 0.063 |
| Hourly speed_6 min after starting | 1.738±0.214 | 2.071±0.405 | 0.334±0.351 | 0.046 | 0.063 |
| CFI_AC_1 min after starting | 2.265±0.582 | 2.806±0.732 | 0.541±0.820 | 0.132 | 0.128 |
| CFI_AC_2 min after starting | 2.303±0.479 | 2.806±0.728 | 0.503±0.660 | 0.090 | 0.091 |
| CFI_AC_3 min after starting | 2.318±0.441 | 2.792±0.722 | 0.475±0.673 | 0.111 | 0.091 |
| CFI_AC_4 min after starting | 2.309±0.438 | 2.787±0.712 | 0.477±0.649 | 0.100 | 0.091 |
| CFI_AC_5 min after starting | 2.319±0.451 | 2.764±0.697 | 0.446±0.645 | 0.117 | 0.128 |
| CFI_AC_6 min after starting | 2.317±0.446 | 2.753±0.686 | 0.436±0.635 | 0.119 | 0.128 |
| CFI _PA_1 min after starting | 5.363±1.236 | 6.243±1.919 | 0.879±1.602 | 0.197 | 0.237 |
| CFI_PA_2 min after starting | 5.351±1.101 | 6.176±1.883 | 0.824±1.351 | 0.158 | 0.176 |
| CFI_PA_3 min after starting | 5.451±0.997 | 6.160±1.882 | 0.709±1.506 | 0.259 | 0.237 |
| CFI_PA_4 min after starting | 5.450±1.002 | 6.177±1.906 | 0.726±1.487 | 0.244 | 0.237 |
| CFI_PA_5 min after starting | 5.475±1.035 | 6.152±1.886 | 0.677±1.463 | 0.267 | 0.237 |
| CFI_PA_6 min after starting | 5.477±1.030 | 6.124±1.860 | 0.648±1.453 | 0.283 | 0.237 |
| CMPB_1 min after starting | 32.180±7.418 | 37.457±11.512 | 5.277±9.611 | 0.197 | 0.237 |
| CMPB_2 min after starting | 32.108±6.609 | 37.053±11.295 | 4.945±8.106 | 0.158 | 0.176 |
| CMPB_3 min after starting | 32.703±5.981 | 36.960±11.293 | 4.257±9.038 | 0.259 | 0.237 |
| CMPB_4 min after starting | 32.701±6.009 | 37.059±11.436 | 4.358±8.924 | 0.244 | 0.237 |
| CMPB_5 min after starting | 32.848±6.209 | 36.912±11.317 | 4.064±8.780 | 0.267 | 0.237 |
| CMPB_6 min after starting | 32.859±6.179 | 36.746±11.159 | 3.887±8.716 | 0.283 | 0.237 |
| CMPB3_1 min after starting | 56.803±5.733 | 53.331±6.442 | -3.473±5.031 | 0.118 | 0.091 |
| CMPB3_2 min after starting | 56.678±5.054 | 52.997±6.078 | -3.681±4.519 | 0.075 | 0.028 |
| CMPB3_3 min after starting | 56.497±4.865 | 52.767±6.223 | -3.729±4.871 | 0.089 | 0.043 |
| CMPB3 _ 4 min after starting | 56.380±4.911 | 52.555±6.201 | -3.825±4.978 | 0.088 | 0.043 |
| CMPB3 _5 min after starting | 56.350±4.835 | 52.491±6.173 | -3.859±4.959 | 0.085 | 0.043 |
| CMPB3 _6 min after starting | 56.397±4.819 | 52.389±6.110 | -4.008±4.955 | 0.076 | 0.043 |

| | | | | | |
|---|---|---|---|---|---|
| The continuous variables are presented as Mean ± SD. ^{a} Paired sample T test, ^{b} No-matrix test (Wilcoxon signed rank test) | | | | | |

As shown in Table 1 above, the 6-minute easy walking test was performed before and after cardiac rehabilitation. The CFI_AC (cardiac force index of walk exercise, the unit is m^{∗}BPM^{-1∗}sec⁻²) and CFI_PA (cardiac force index of peak acceleration from walk, the unit is m^{∗}BPM^{-1∗}sec⁻²) are both the methods to detect the cardiac state during physical exercise, which has obtained both the US and Taiwan patents, can be used as the reference data in verifying CMPB and CMPB3. From Table 1, we can see that after cardiac rehabilitation, both CF1_AC and CFI_PA were increased, indicating an improvement in cardiac status. It can also be seen from Table 1 that the CMPB of this invention has increased significantly, and the state of heart failure has improved from the state of the third and fourth grades to the state of the first and second grades and is improving progressively. The CMPB3 of this invention approaches to 50, which shows that cardiac rehabilitation is indeed effective, and is also proved that CMPB (positive correlation with CF1_AC and CFI_PA) and CMPB3 in this invention can indeed evaluate the cardiac state.

From Table 2 below, the correlation between CF1_AC and the maximum oxygen consumption (VO2 max) shows a positive correlation. From Table 3 below, the correlation between CMPB and the anaerobic threshold (AT) shows a positive correlation. From Table 4 below, the correlation between CMPB and the oxygen pulse (O2 pulse) also shows a positive correlation.

**Table 2. Correlation between maximum oxygen consumption and CF1_AC measured before cardiac rehabilitation**

| Pre-rehabilitation | (1) | (2) | (3) | (4) | (5) | (6) | (7) | (8) |
|---|---|---|---|---|---|---|---|---|
| VO₂ max(1) | 1.000 | | | | | | | |
| VO₂ max%(2) | 0.772^{∗∗} | 1.000 | | | | | | |
| CFI_AC_1 min after starting (3) | 0.377^{∗} | 0.222 | 1.000 | | | | | |
| CFI_AC_2 min after starting (4) | 0.407^{∗} | 0.293 | 0.988^{∗∗} | 1.000 | | | | |
| CFI_AC_3 min after starting (5) | 0.379^{∗} | 0.250 | 0.977^{∗∗} | 0.991^{∗∗} | 1.000 | | | |
| CFI_AC_4 min after starting (6) | 0.368^{∗} | 0.243 | 0.968^{∗∗} | 0.986^{∗∗} | 0.997^{∗∗} | 1.000 | | |
| CFI_AC_5 min after starting (7) | 0.359 | 0.238 | 0.967^{∗∗} | 0.984^{∗∗} | 0.996^{∗∗} | 0.999^{∗∗} | 1.000 | |
| CFI_AC_6 min after starting t (8) | 0.367^{∗} | 0.247 | 0.967^{∗∗} | 0.984^{∗∗} | 0.995^{∗∗} | 0.998^{∗∗} | 0.999^{∗∗} | 1.000 |

| | | | | | | | | |
|---|---|---|---|---|---|---|---|---|
| Spearman's rank correlation coefficient ^{∗}p<0.05: ^{∗∗}p<0.01 | | | | | | | | |

**Table 3. Correlation between Anaerobic Threshold (AT) and displacement per heartbeat measured before cardiac rehabilitation**

| Pre-rehabilitation | (1) | (2) | (3) | (4) | (5) | (6) | (7) | (8) | (9) |
|---|---|---|---|---|---|---|---|---|---|
| AT(1) | 1.000 | | | | | | | | |
| AT (%) Predicted VO₂max(2) | 0.779^{∗∗} | 1.000 | | | | | | | |
| AT(ml/kg/min(3) | 0.830^{∗∗} | 0.673^{∗∗} | 1.000 | | | | | | |
| CMBP_1 min after start (3) | 0.239 | 0.331 | 0.145 | 1.000 | | | | | |
| CMBP_2 min after start (4) | 0.304 | 0.430^{∗} | 0.221 | 0.974^{∗∗} | 1.000 | | | | |
| CMBP_3 min after start (5) | 0.320 | 0.440^{∗} | 0.232 | 0.966^{∗∗} | 0.992^{∗∗} | 1.000 | | | |
| CMBP_4 min after start (6) | 0.328 | 0.426^{∗} | 0.219 | 0.953^{∗∗} | 0.984^{∗∗} | 0.994^{∗∗} | 1.000 | | |
| CMBP_5 min after start (7) | 0.310 | 0.425^{∗} | 0.219 | 0.957^{∗∗} | 0.985^{∗∗} | 0.993^{∗∗} | 0.997^{∗∗} | 1.000 | |
| CMBP_6 min after start (8) | 0.316 | 0.434^{∗} | 0.220 | 0.956^{∗∗} | 0.984^{∗∗} | 0.992^{∗∗} | 0.996^{∗∗} | 0.999^{∗∗} | 1.000 |

| | | | | | | | | | |
|---|---|---|---|---|---|---|---|---|---|
| Spearman's rank correlation coefficient ^{∗}p<0.05: ^{∗∗}p<0.01 AT(%) Predicted VO₂max: Percentage (%) of anaerobic threshold 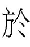 maximum oxygen consumption | | | | | | | | | |

**Table 4. Correlation between O₂ pulse and displacement per heartbeat measured before cardiac rehabilitation**

| Pre-rehabilitation | (1) | (2) | (3) | (4) | (5) | (6) | (7) | (8) |
|---|---|---|---|---|---|---|---|---|
| O₂ pulse (1) | 1.000 | | | | | | | |
| % of O₂ pulse (2) | 0.648^{∗∗} | 1.000 | | | | | | |
| CMBP_1 min after start (3) | 0.412^{∗} | 0.257 | 1.000 | | | | | |
| CMBP_2 min after start (4) | 0.427^{∗} | 0.300 | 0.974^{∗∗} | 1.000 | | | | |
| CMBP_3 min after start (5) | 0.430^{∗} | 0.279 | 0.966^{∗∗} | 0.992^{∗∗} | 1.000 | | | |
| CMBP_4 min after start (6) | 0.438^{∗} | 0.270 | 0.953^{∗∗} | 0.984^{∗∗} | 0.994^{**} | 1.000 | | |
| CMBP_5 min after start (7) | 0.417^{∗} | 0.256 | 0.957^{∗∗} | 0.985^{∗∗} | 0.993^{∗∗} | 0.997^{∗∗} | 1.000 | |
| CMBP_6 min after start (8) | 0.418^{∗} | 0.250 | 0.956^{∗∗} | 0.984^{∗∗} | 0.992^{∗∗} | 0.996^{∗∗} | 0.999^{∗∗} | 1.000 |

| | | | | | | | | |
|---|---|---|---|---|---|---|---|---|
| Spearman's rank correlation coefficient ^{∗}p<0.05: ^{∗∗}p<0.01 | | | | | | | | |

Furthermore, the evaluating method of the cardiac load and the evaluating device of this invention can be combined with the application program of the mobile devices. It allows users to know their cardiac load level in real time through the app, to adjust the exercise method and intensity to maintain the exercise safety.

Furthermore, the device for evaluating the cardiac load of this invention can also be used in military training, so that the trainer can know the degree of the cardiac load of each trainee, and thereby adjust the training method and intensity of the trainee.

Therefore, this invention is innovative, progressive, and available for industrial use. It should undoubtedly meet the patent application requirements of the ROC Patent Act. We hereby file the patent application in this invention according to the law and anticipate the Authority's patent granting.

However, the above are only preferred embodiments of this invention and are not used to limit the scope of implementation of this invention. For example, all shapes, structures, features, and spirits described in the scope of the patent application of this invention are equal changes and modifications shall be included in the scope of the patent application of this invention.

## Claims

1. A method for evaluating cardiac load, the steps comprise:
measuring a heartbeat number of a test specimen within a period with a heartbeat rate detection unit, and measuring a displacement of the test specimen within the period with a distance measuring unit;
the heartbeat rate detection unit and the distance measuring unit transmit the heartbeat number and the displacement to an operation unit respectively;
the operation unit obtains a displacement per heartbeat according to the heartbeat number and displacement, the calculation is: the displacement per heartbeat equal to the displacement divided by the heartbeat number;
when the displacement per heartbeat is greater than a first preset value or less than a second preset value, the operation unit transmits a warning message and the displacement per heartbeat to a display unit; and
the display unit displays the warning message and the displacement per heartbeat, and warns the test specimen;
wherein the heartbeat number is the heartbeats per second, the heartbeat rate detection unit measures the heartbeat number of the test specimen by measuring the heartbeats or arterial beats of the test specimen.

2. The method for evaluating cardiac load of CLAIM 1, in which in the step that the operation unit obtains a displacement per heartbeat according to the heartbeat number and the displacement, the calculation is: the displacement per heartbeat equal to the displacement divided by the heartbeat number, it also comprise the following steps:
the operation unit obtains an hourly speed according to the displacement and the period, and obtains a normalized displacement per heartbeat within 3-km distance according to the displacement per heartbeat and the hourly speed, the calculation is: the normalized displacement per heartbeat within 3-km distance equal to the displacement per heartbeat multiplying (3 divided by the hourly speed);
the operation unit transmits the normalized displacement per heartbeat within 3-km distance to the display unit; and
the display unit displays the normalized displacement per heartbeat within 3-km distance.

3. A device for evaluating cardiac load, which comprises:
a heartbeat rate detection unit used to measure a heartbeat number of a test specimen;
a distance measuring unit used to measure a displacement of the test specimen;
an operation unit used to receive the heartbeat number and the displacement, and calculate a displacement per heartbeat according to the heartbeat number and the displacement, the calculation is: the displacement per heartbeat equal to the displacement divided by the heartbeat number; and
a display unit used receive the displacement per heartbeat sent by the operation unit and to display the displacement per heartbeat;
wherein when the displacement per heartbeat is greater than a first preset value or less than a second preset value, the operation unit further transmits a warning message to the display unit.

4. The device for evaluating cardiac load of CLAIM 4, in which the operation unit further obtains an hourly speed according to the displacement and the period, and a normalized displacement per heartbeat within 3-km distance according to the displacement per heartbeat and the hourly speed, the calculation is: the normalized displacement per heartbeat within 3-km distance equal to the displacement per heartbeat multiplying (3 divided by hourly speed),and the operation unit further transmits the normalized displacement per heartbeat within 3-km distance to the display unit displaying the normalized displacement per heartbeat within 3-km distance.

5. The device for evaluating cardiac load of CLAIM 3 or CLAIM 4, in which the evaluating device further comprises a positioning unit used to locate and obtain the position of the test specimen.

6. A method for evaluating cardiac load, the steps comprise:
measuring a heartbeat number of a test specimen within a period with a heartbeat rate detection unit, and measuring a displacement of the test specimen within the period with a distance measuring unit;
the heartbeat rate detection unit and the distance measuring unit transmit the heartbeat number and the displacement to an operation unit respectively;
the operation unit obtains a maximum oxygen consumption according to the heartbeat number and the displacement, the calculation is: the maximum oxygen consumption equal to the displacement divided by (the heartbeat number multiplying K), wherein 0.71≦K≦3.63;
when the maximum oxygen consumption is greater than a third preset value or less than a fourth preset value, the operation unit transmits a warning message and the maximum oxygen consumption to a display unit; and
the display unit displays the warning message and the maximum oxygen consumption and warns the test specimen;
wherein the heartbeat number is the heartbeats per second, the heartbeat rate detection unit measures the heartbeat number of the test specimen by measuring the heartbeats or arterial beats of the test specimen.

7. A device for evaluating cardiac load, which comprises:
a heartbeat rate detection unit used to measure a heartbeat number of a test specimen;
a distance measuring unit used to measure a displacement of the test specimen;
an operation unit used to receive the heartbeat number and the displacement, and calculate a maximum oxygen consumption according to the heartbeat number and the displacement, the calculation is: the maximum oxygen consumption equal to the displacement divided by (the heartbeat number multiplying K), where 0.71≦K≦3.63; and
a display unit used to receive the maximum oxygen consumption sent by the operation unit and to display the maximum oxygen consumption;
wherein when the maximum oxygen consumption is greater than a third preset value or less than a fourth preset value, the operation unit further transmits a warning message to the display unit.

8. The device for evaluating cardiac load of CLAIM 7, in which the evaluating device further comprises a positioning unit used to locate and obtain the position of the test specimen.

9. A method for evaluating cardiac load, the steps comprises:
measuring a heartbeat number of a test specimen within a period with a heartbeat rate detection unit, and measuring a displacement of the test specimen within the period with a distance measuring unit;
the heartbeat rate detection unit and the distance measuring unit transmit the heartbeat number and the displacement to an operation unit respectively;
the operation unit obtains an anaerobic threshold according to the heartbeat number and the displacement, the calculation is: the anaerobic threshold equal to the displacement divided by (the heartbeat number multiplying M), wherein 20.5≦M≦112.07;
when the anaerobic threshold is greater than a fifth preset value or less than a sixth preset value, the operation unit transmits a warning message and the anaerobic threshold to a display unit; and
the display unit displays the warning message and the anaerobic threshold and warns the test specimen;
wherein the heartbeat rate is the heartbeats per second; the heartbeat rate detection unit measures the heartbeat number of the test specimen by measuring his/her heartbeats or arterial beats.

10. A device for evaluating cardiac load, which comprises:
a heartbeat rate detection unit used to measure a heartbeat number of a test specimen;
a distance measuring unit used to measure a displacement of the test specimen;
an operation unit used to receive the heartbeat number and the displacement and calculate an anaerobic threshold according to the heartbeat number and the displacement, the calculation is: the anaerobic threshold equal to the displacement divided by (the heartbeat number multiplying M), wherein 20.5≦M≦112.07; and
a display unit used to receive the anaerobic threshold sent by the operation unit, and to display the anaerobic threshold;
wherein when the anaerobic threshold is greater than a fifth preset value or less than a sixth preset value, the operation unit further transmits a warning message to the display unit.

11. The device for evaluating cardiac load of CLAIM 10, in which the evaluating device further comprises a positioning unit used to locate and obtain the position of the test specimen.

12. A method for evaluating cardiac load, the steps comprise:
measuring a heartbeat number of a test specimen within a period with a heartbeat rate detection unit, and measuring a displacement of the test specimen within the period with a distance measuring unit;
the heartbeat rate detection unit and the distance measuring unit transmit the heartbeat number and the displacement to an operation unit respectively;
the operation unit obtains an oxygen pulse according to the heartbeat number and the displacement, the calculation is: the oxygen pulse equal to the displacement divided by (the heartbeat number multiplying N), wherein 1.36≦N≦5.58;
when the oxygen pulse is greater than a seventh default value or less than an eighth default value, the operation unit transmits a warning message and the oxygen pulse to a display unit; and
the display unit displays the warning message and the oxygen pulse and warns the test specimen;
wherein the heartbeat number is the heartbeats per second, and the heartbeat rate detection unit measures the heartbeat number of the test specimen by measuring the heartbeats or arterial beats of the test specimen.

13. A device for evaluating cardiac load, which comprises:
a heartbeat rate detection unit used to measure a heartbeat number of a test specimen;
a distance measuring unit used to measure a displacement of the test specimen;
an operation unit used to receive the heartbeat number and the displacement, and calculate the oxygen pulse according to the heartbeat number and the displacement, the calculation is: the oxygen pulse equal to the displacement divided by (the heartbeat number multiplying N), where 1.36≦N≦5.58; and
a display unit used to receive the oxygen pulse sent by the operation unit and to display the oxygen pulse;
wherein when the oxygen pulse is greater than a seventh preset value or less than an eighth preset value, the operation unit further transmits a warning message to the display unit.

14. The device for evaluating cardiac load of CLAIM 13, in which the evaluating device further comprises a positioning unit used to locate and obtain the position of the test specimen.
